# EUROPEAN PATENT SPECIFICATION

(11) **EP 0 847 404 B1**
(45) Date of publication and mention of the grant of the patent: **19.05.1999**
(21) Application number: 96926572.7
(22) Date of filing: 09.08.1996
(51) Int. Cl.: C08F 2/00, C07C 313/12, C07C 309/08

(54) **NON POLLUTING ANTIFOULING MATERIAL FOR COATING POLYMERIZATION VESSELS**
UMWELTFREUNDLICHES VERKRUSTUNGABWEISENDES MATERIAL ZUR BESCHICHTIGUNG VON POLYMERISATIONSREAKTOREN
MATERIAU ANTISALISSURE NON POLLUANT POUR LE REVETEMENT DES REACTEURS DE POLYMERISATION

(30) Priority: 29.08.1995 IT UD950165
(43) Date of publication of application: 17.06.1998
(73) Proprietor: C.I.R.S. S.P.A., 45010 Rovigo (IT)
(72) Inventor: CARLIN, Francesco, I-45010 Rovigo (IT); SATTIN, Mario, I-45010 Rovigo (IT)
(74) Representative: D'Agostini, Giovanni, Dr.
(86) International application number: IT9600161
(87) International publication number: WO9708210

(56) References cited:
- EP-A- 0 091 965
- EP-A- 0 096 319
- FR-A- 2 378 045
- FR-A- 2 535 325
- US-A- 4 142 033

## Description

### Technical field:

The object of this invention is a non polluting antifouling material for coating polymerization vessels.

It is known that the process for obtaining a polymer is carried out in reactors known as polymerization vessels.

It is also known that during the polymerization, notable amounts of material being polymerized deposit on the walls of the reactor and on the respective pipes, forming a polluting layer which hinders heat exchange and clogs pipes, valves, etc.

For this reason, for over twenty years antifouling materials, for preventing the deposit of the material being polymerized on the walls of the reaction vessel and on the pipes, have been used.

This invention regards these antifouling materials.

### Background art

Despite the use of different kinds of antifouling agents, problems due to the formation of deposits still exist, having as main drawbacks:
- considerable maintenance works for removing the deposits with subsequent interruption of the production, opening of the reactor and therefore immission in the atmosphere of large amounts of gaseous monomers with serious pollution of the environment;
- pollution of the resulting product, since some fouling parts (of very dark colour) finish inside the polymerized material produced, with subsequent lower quality and complaints from the users;
- very serious ecological problems for the elimination of the deposits as waste.
- The particles that detach from the layer are of a bluish or dark brown colour tending to black, contaminate the obtained polymers in the form of black specks, that worsen the qualitative aspect of the finished product. In prior art, the use of antifouling material has been developed with greater attention in the production of polyvinylchloride in water suspension.

Among the various substances used as antifouling agents, those of greater interest were obtained through the technique foreseeing the condensation of polyarylphenols with formaldehyde.

For this purpose we refer to:
- US - 3.669.946 (filed in U.S. on August 31, 1970, and disclosed on June 13, 1972) which describes the general concept behind the use of polar organic substances, such as for example naphthenic dyes, formaldehyde, alphanaphthelamine, nygrosine and many others.
- US - 3.825.434 published on July 27, 1974, which describes an antifouling agent obtained from the condensation of phenol with formaldehyde. It has been proved that this antifouling material is not very effective for preventing the deposits on the walls, seemingly because the formaldehyde phenol condensate has a lattice structure.
- EP 0052421 describes a process for obtaining an antifouling agent which differs from the previous ones for the fact that phenol is replaced with 1-naphthol (α-naphthol) in which the nuclear positions 2 and 4 are not substituted and the nuclear position 3 is not substituted or has a substituent which is not strongly electron-attracting.

FR,A,2 535 325 (TOYO SODA MANUF. CO., LTD) 4 May 1984: Discloses a process for non-acqueous polymerisation of vinyl chloride utilising a -SO3 Na radical.

This product gives a strongly coloured solution clearly not acceptable in the process because it results in a pollutant colour. Furthermore, this product has a low adhesive capacity on the internal surface of the reactor and is easily removable by the monomer of polyvinylchloride.

EP,A, 0 096 319 (SHIN-ETSU CHEM. CO., LTD) 21 December 1983: Discloses a method for preventing scale deposition in the polymerization of ethylenically unsaturated monomers. The method comprises coating the reactor walls in advance with an acqueous coating composition comprising an organic dye of sulfonic or carboxylic acid type in the form of an alkali metal or ammonium salt and an acqueous colloidal dispersion of an inorganic material and then drying the coated surface.

This solution has the same defects as the previous one.

EP,A,0 091 965 (SHIN-ETSU CHEM. CO., LTD) 26 October 1983:

Discloses a process for preventing deposition of polymer scale during polymerization of vinyl monomer, wherein an acqueous coating solution containing an alkali metal or ammonium salt of sulfonic acid type or carboxylic acid type dye or organic sulfonic or carboxylic acid having at least a pair of conjugated double bonds per molecular and polyvinyl alcohol in a specific proportion and having a pH not exceeding 7 on the wall of a reactor prior to conducting the polymerization.

This solution has the same defects as the previous ones and furthermore has no efficiency in preventing crust build-up during polymerization on the inner wall of the reactor and is not suitable for products that come in contact with foodstuffs, eg mineral water plastic bottles.

US,A,4, 142 033 (D.WITENHAFER) 27 February 1979:

Discloses an inversion polymerization process for producing vinyl resins by an inversion polymerization technique in the presence of a two-layer coating on the internal surfaces of the polymerization reactor, wherein it is provided to use dyes to make the prime coating which contain in their chemical structure one or more of -SO3H and/or -SO3Na radicals.

This product is strongly coloured and obliges the operator to adopt techniques not suitable for PVC medical and food applications and having the same defects as the previous one and furthermore, is not able to work well at the polymerization temperature of PVC's between 65°C - 75°C.

These antifouling products are of a dark brown or blue colour tending to black; moreover, it has been observed that some of these products have very low efficiency at high polymerization temperatures, and that other ones, when in contact with the oxygen of the air present in the polymerization vessels do not give an effective protection and easily degrade in an irreversible manner.

The aim of this invention is to avoid the above-mentioned drawbacks and to eliminate the above-mentioned defects.

### Disclosure of invention

The first purpose was to make the pre-existing dark antifouling agents colourless, so that the particles of PVC crusts, which came off from the walls of the reactor during polymerization and/or during the rinsing, would not contaminate the finished product and the fouling to be recovered.

Taking into account the technique described in patent USA 2.848.436 published on August 19, 1958, which claims the preparation of products of the condensation of formaldehyde with colourless alkenyl-phenols by using sodium hydrosulphite, it was thought to utilize the same principle for preparing decolourized antifouling agents.

However, as the pre-existing antifouling agents were condensed to maximum lattice, it was not possible to utilize said technique since only a weak decoloration occurred, the colour passing from bluish-black to dark green, and, above all, a great amount of salts precipitated, indicating that the reaction had not taken place.

To reach the first positive results it was necessary to find a product obtained under particular temperature and time conditions, for which the lattice degree was not as great and which would still allow the reaction with sodium hydrosulphite.

In particular, it has been observed that the product deriving from the condensation of formaldehyde with alphanaphthol was that which gave the best modification possibilities.

However, the results were not constant because the different and violent reactivity of formaldehyde made it difficult to control the reaction and the times of intervention.

It was thought to find a solution also for this last drawback by replacing formaldehyde, the cause of the uncontrollable and irreversible lattice, with the following type of reagents:
a - sodic salt of hydroximethansulphinic acid;
b - sodic salt of hydroximethansulphonic acid;
c - the product of the reaction between sodium hydrosulphite and formaldehyde prepared separately.

By carrying out the reaction in the absence of oxigen and under particular time and temperature conditions, subsequently described in the enclosed examples, and by using derivatives of sulphur, the desired decolouration results were obtained.

The alkaline water solution of these products, at pH 12.5 was perfectly clear and had a light yellow colour, which, in the presence of oxygen, became blue; in its absence the solution rapidly changed to its original colour.

The phenomenon can be easily repeated for a large number of samples.

In the tests of polymerization carried out on industrial reactors and described in the enclosed examples, the decolourized (colourless or transparent) non polluting antifouling agent previously added with polyvinylic alcohol, formed on the walls of the reactors a thin film which appeared to be very resistant to abrasion and, above all, with very great antifouling properties.

From the analyses subsequently carried out, it appears that the new product has a completely new molecular structure if compared to that of the pre-existing antifouling agents, and that it has an extraordinary resistance against the tacking of the material being polymerized in the reactor; it does not come off from the wall, and, lasting longer, wears off more slowly, and, furthermore, only a very thin layer is required on the wall, therefore the amount of material used is less.

The reasons of such a surprising effect were sought, and the only factor which can justify this surprising discovery, is the massive presence of sulphur, which not only explicates a decolouring function, but also acts as a binder for macro-molecules, as a strong stabilizer of the molecules obtained, placing itself in any free position of the naphthenic group, stabilizing it and making it into a strong repeller, as it notoriously is in rubbers.

The product therefore has not only simple decolouration properties, but also new and relevant non-stick ones, and thus it can be considered a much greater innovation if compared to the initial aim, in particular if one takes into account the great and serious ecological problems still existing that present-day polymerization processes of vinyl chloride, styrene, acrylonitrile, butadiene, acrylic acid, polyacetate and many of their copolymers, cause.

Despite the enormous investments in studies and research for solving the problem, up to now no satisfactory results had been obtained.

In order to demonstrate the importance of the present invention, the inventors, knowing that the tests carried out on small laboratory or pilot reactors were not representative for the known reasons (different types of agitation, different surface-volume ratioes and above all to the non uniform application on the internal surfaces of the reactor), had the possibility, thanks to the collaboration of important chemical industries interested in exploiting this interesting discovery, to test the new non polluting antifouling material for long periods of time on industrial production reactors.

### Structure of the new product

From subsequent analyses it has been discovered, as claimed, that the main antifouling action of the product, is given by the inclusion of sulphur in the radicals of the naphthenic structure.

The problem has been therefore solved with a new antifouling material, which is applied as a protective coating to the inner walls of a polymerization vessel, of the type that includes aromatic molecules of the naphthenic type with radicals in position from 1 to 8, according to the arrangement: 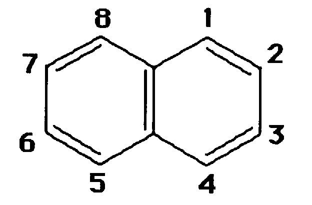 wherein it includes at least one atom of sulphur (S) in at least one of these radicals.

In the antifouling material there is a substantial presence of sulphur and the agent is characterized by the presence of sulphur which does not represent an impurity; therefore its contents must be higher than 0.25% in weight, preferably higher than 0.85%, the optimal percentage being 9.3%.

Advantageously, the structure of sulphur can be bound from the radical to the oxygen (O).

Just as advantageously, the radical is characterized by the presence of SOₙ where n may be 2 or 3.

In the optimal solution the radical is characterized by the presence of the SOₙNa group, where n may be 2 or 3.

From the tests and the spectroscopical examinations and from the analyses on the products, it has been verified that the greatest activity is obtained by adding to a naphthenic structure, sulphonic and sulphinic radicals salified through the presence of sodium in the substantial formulation -CH₂SOₙNa which can be found as a single radical in positions 2,3 or 4 of the naphthenic structure, or in the formulation -CH(SOₙNa) - if it binds two of these structures.

This feature as claimed being identified in the present invention and being able to result in a liquid product, perfectly transparent and colourless in the absence of oxygen, becoming progressively dark in the presence of oxygen and returning colourless if restored in the absence of oxygen, and resulting really strongly effective in performance as antifouling, as the scope of the invention.

In the product can co-exist different molecules
A. First type of molecule, simple: 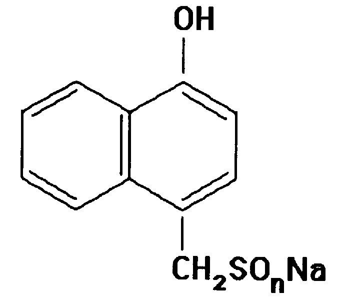 Where n can vary from 2 to 3.
B. Second type of molecule, simple: 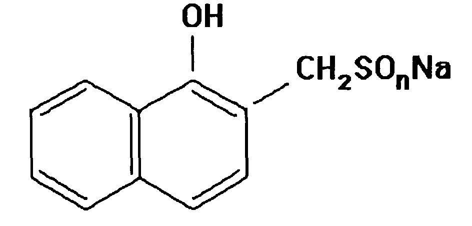 Where n can vary from 2 to 3.
C. Third type of molecule, simple: 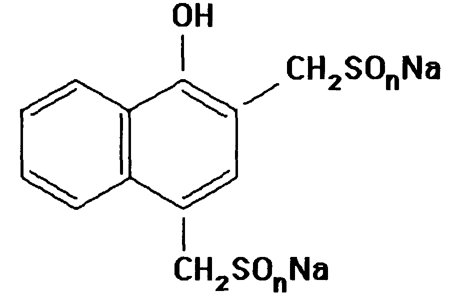 Where n can vary from 2 to 3.
D. Fourth type of molecule, more complex, binding two naphthenic structures: 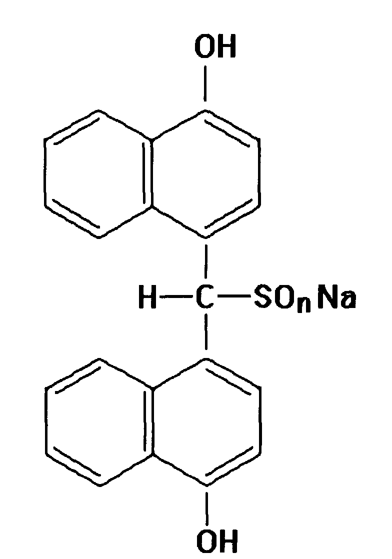 Where n can vary from 2 to 3.
E. Fifth type of molecule, even more complex, binding two naphthenic structures or reaction intermediate: 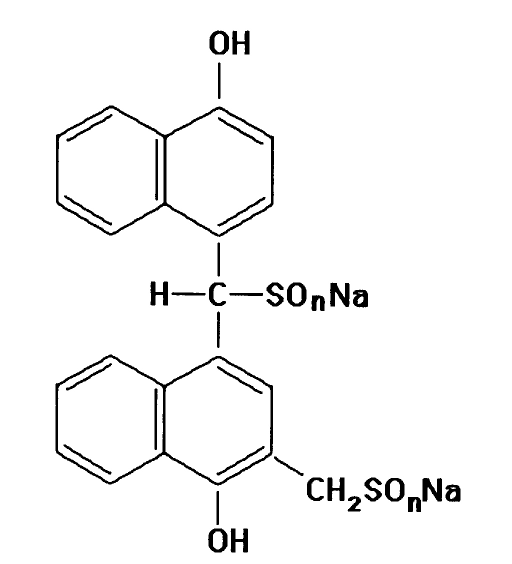 Where n can vary from 2 to 3.
F. Sixth type of molecular structure, an even more complex reaction intermediate: 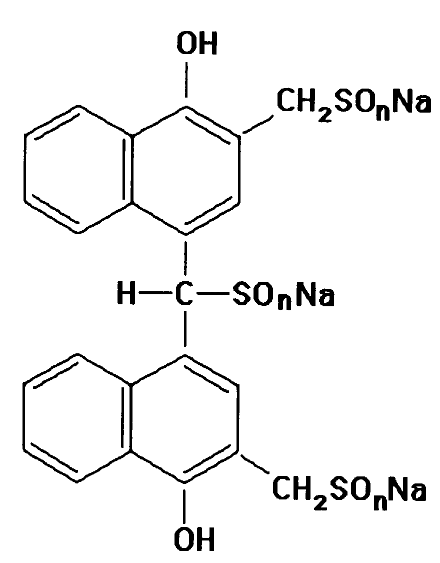 Where n can vary from 2 to 3.

The new product is therefore characterized by the contemporary presence of these molecules, with one or the other prevailing according to the reaction conditions.

### Formation of the new product:

The product to which the present invention refers, is obtained by causing the reaction of a product having naphthenic (aromatic) structure, such as for example of the alphanaphthol type, with the sodic salt of hydroximethansulphinic acid having the empirical formula CH₃NaO₃S and the following structural formula:

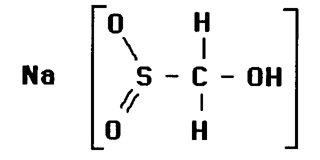

The sodic salt of hydroximethansulphinic acid reacts with alphanaphthol in the weight ratio of 1 to 1.5, in a 10 to 50% water solution and bringing the solution to a temperature between 40° and 100°C in nitrogen atmosphere and in an alkaline environment (pH 11-13), forming the new product according to the present invention.

The solution of the product thus condensed has a light transparent aspect, and if left for a certain period in the presence of air (oxygen) it slightly oxidizes, turning to a bluish colour but, if the contact with oxygen is interrupted, the product turns back to its original light transparent aspect.

This physical behaviour proves that a completely new product has been synthesised, which is structurally different from those of the previous techniques, in which this physical phenomenon could not be observed.

Various hypotheses have been made on the structural nature of the product, and although not being entirely certain of the causes for this reversibility, it is thought that the phenomenon can be related to the presence in the condensate of the claimed sulphinic or sulphonic radicals.

The reaction that takes place is substantially the following:

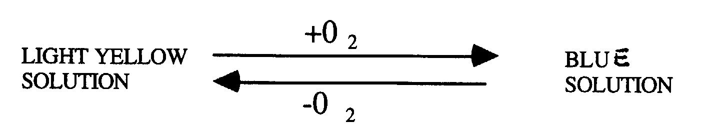

This characteristic further distinguishes the new product from those obtained with previous techniques.

The structure of the resulting condensate, as proved, is very different and innovative not only for the presence of the sulphur atom (S) in the respective radicals but also for the great reactivity and lability of the molecules thus formed.

### Other possible types of realisation

The product may also be made by replacing the sodium salt of hydroxymethansulphinic acid with the sodium salt of hydroxymethansulphonic acid.

In a more complex and expensive way, one can also make formaldehyde react with sodium hydrosulphite and therefore the resulting product with a naphthenic one, such as for example α-naphthol.

The reaction presumably occurs as follows:

The sodium salt of hydroxymethansulphinic acid reacts with α-naphthol or with its sodium salt, forming the carbocation: 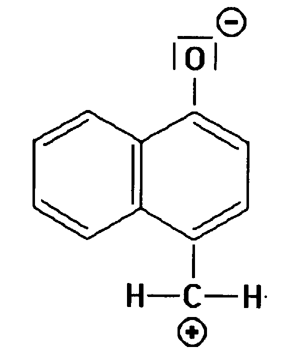 or its isomer: 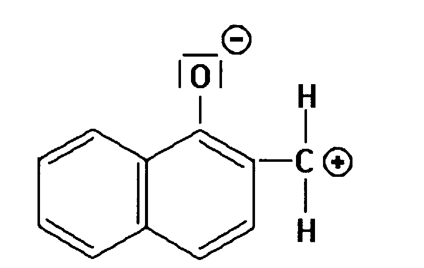 giving as final result the structures previously named A, B, C, D, E and F.

### Application tests

The product applied to the surface of the reactor in an atmosphere deprived of oxygen, once dry, has a light yellow-brown colour, contrary to the dark navy colour tending to black of the antifouling agents used at present.

In order to maintain not only the transparent aspect but also its efficiency, the antifouling material in the liquid state is kept in airtight containers.

The best preservation is obtained by pressurizing the container with inert gas, preferably nitrogen.

The best containers are those in glass or, better still, in polyethylene terephthalate (PET), so that the containers will not pollute the environment and can be completely recycled.

The simplest shape is that of bottles that are commonly used for fizzy mineral water.

The product thus conserved remains unaltered, having a pale colour slightly tending to yellow or ivory yellow, and in the tests for the application on the walls of the reactor, the antifouling material deposits on the wall forming a thin layer which becomes, as previously mentioned, of a light yellow-brown colour.

The application on the walls of the reactor is carried out in the absence of oxygen by spraying it with water vapour at high temperature.

The higher the temperature, the greater the adherence of the wall coating.

Up to now the application tests had only been carried out on small laboratory reactors with a volume of about 1 litre.

The results in these small reactors were always positive, even with low quality antifouling materials, and this was due to the simple fact that the thickness of the coating is always the same, but the volume/thickness ratio of the coating is remarkably different in a laboratory reactor and in those used for the production.

In fact, a production reactor may have a volume of 100,000 litres or more.

The coating applied on the wall has practically always the same thickness, for example a few tenths of a millimeter.

Therefore the results of a coating of equal thickness on reactors of totally different volume, which in the case described in the present invention had a ratio of 1 to 100,000, undoubtedly cannot be considered identical.

For this reason, tests were directly carried out on production reactors, as described in the following pages.

It has been calculated that, in order to maintain a 136 m³ reactor clean, with working cycles exceeding 1000 loads, about 6 LT. of water solution, prepared as described in example 1 and sprayed after each load, are necessary.

Taking into account that in 6 LT. of a 5% active principle solution only 300 grammes of antifouling agent are present, and that of these, only 30 grammes form the protective layer coating the inner parts of the reactor, while the remaining 270 are recovered in the condensate outlet after the spraying with vapour, it can be seen that the amount of the antifouling agent object of this invention is infinitely low if compared to the 45,000 Kg. of monomers loaded.

In fact, thanks to the minimal amount of product that can come into in contact of the finished polymers, it has never been possible with the analytical means presently in use, to detect traces of product in the polymers and in their finished products.

This has been confirmed by the various analytical tests carried out in order to ask health authorities for the authorization to use the antifouling agent in the production of polymers for making food and medical containers.

It has moreover been observed that the action of this new antifouling agent occurs in two different stages, and more precisely:
- a first period of application, that can range from 100 to 400 loads, which requires the amounts of antifouling agent indicated in the enclosed examples.

In this way, the previous polymers or the other types of antifouling agent occluding the micropores of the reactor's surfaces which also acted as support for further deposits, were perfectly removed.
- a second and final application cycle where the necessary amounts of antifouling agent are halved or even reduced to 1/4, since the micropores of the inner surfaces of the reactor are already occluded and well protected by an optimal quantity of the antifouling agent object of the present invention.

The inside of the reactor, after 4 months of discontinuous running (2.5 loads per day), had a light yellow brown colour, with no trace of polymers and in particular having the upper parts in contact with the gas monomer phase (reflux condensers, safety valves and flow piping) perfectly clean which did not require any type of cleaning.

This gradual decrease in the amounts of antifouling agent utilized, allows, on the contrary of the prior techniques, to supply the rinse of the reactor to the tanks for collecting the water suspensions of the obtained polymer, without further waste discharges, and therefore allowing a complete and effective, and above all cheap, load and unload technology of the polymerization vessels, with a technique known by the experts of the sector as:
"closed man-hole".

As the sodic salt of hydroximethansulphinic acid may also be prepared with a molar excess of hydrosulphite, also by using this product a condensate of analogous or improved performance (a greater reducing effect) would be obtained.

### Examples of preparation and application

### Example 1: Preparation of the non polluting antifouling agent.

In a 8000 litre stainless steel reactor, equipped with an anchor agitator with speed ranging from 20 to 40 rpm, Kg. 1200 of water, Kg. 180 of a 30% NaOH solution, Kg. 270 of alphanaphthol are respectively loaded under a rigorous nitrogen flow.

The temperature is brought to 90 °C, and in 2 hours Kg.900 of a 31,5% w/w sodium hydroximethansulphinate water solution is percolated. The solution is kept at 90°C for 12 hours and then Kg. 190 of a 30% NaOH solution are added.

At the end of the reaction, a perfectly transparent yellow water solution is obtained, to which Kg. 800 of a 4% polyvinylic alcohol water solution was added, having a viscosity at 20°C of 45 cP and with a hydrolysis degree exceeding 99% OH.

The mass was cooled and then diluted with water until a final percentage of solids equal to 5% was reached.

The solution thus obtained was transferred into 1.5 LT PET bottles under a nitrogen flow.

The bottles were then ready to be used in antifouling tests in industrial production reactors.

A. From the laboratory analysis carried out on the non polluting antifouling agent water solution, the following results were obtained:
A1. % Solid matter in a stove at 120°C for 3 hours: 5%.
A2. pH = 12.3
A3. Specific weight at 20°C: 1.028
A4. Reversibility of the product in contact with air at room temperature

- The 1.5 LT. PET bottle is opened and a flow of air is introduced for 5 seconds.
- The bottle is closed and subjected to agitation for 1 minute.
- The colour of the solution changes from light yellow to dark blue.
- The bottle, after 5 minutes, shows that vacuum has been formed inside it, and the colour of the liquid, after about 60 minutes, changes once again from dark blue to light yellow.

This test indicates that the product has absorbed all the oxygen supplied and that it is still active.

### B. Analyses on the product desiccated as such

By evaporating the water of the solution at 60°C in a nitrogen environment, a colourless product is obtained in the form of a slightly yellow powder that is subjected to various analyses:

### B1. Elementary analysis

The elementary analysis on the product, carried out with the Carlo Erba instrument model EA 1108, for the determination of carbon, hydrogen, nitrogen, sulphur, and by means of atomic absorption spectroscopy (AAS) for the determination of sodium, gave the following results:
- Carbon: 55.6%
- Hydrogenized: 3.84%
- Nitrogen: < 0.10%
- Oxygen: 15.86%
- Sulphur: 8.8%
- SODIUM 15.8%

### B2. Spectrophotometrical analysis

An infrared spectrophotometer was used for analyzing the product prepared by following the technique of the pellet in KBr at a concentration of 0.1% in weight.

The instrument used was a Perkin-Elmer FT-IR model 7200.

The spectrum showed the presence of bands characterizing the groups presumed in the description of the product's structure and, in particular, the bands at 970 cm⁻¹, 640 cm⁻¹ and 500 cm⁻¹.

### Example 2: Polymerization tests of polyvinylchloride in suspension (PVC-S) on large industrial reactors

In order to verify the concrete efficiency of the new antifouling agent prepared as described in example 1, a large reactor was chosen, having the volume of 136 m³. This vessel is used in the production of K-57, a delicate type of PVC-S, having low molecular weight and therefore suitable for the production of bottles or containers (injection and blowing) for mineral water and other products for medical use.

For various years, an antifouling agent, produced according to the known techniques described in the introduction of the present patent, was used in this reactor, and therefore for many years the production had been optimized with the following parameters reported for each batch:
a - washing of the reactor with high pressure water (> 200 bar).
b - application of the antifouling agent with about 200 LT. of solvent.
c - heating of the walls with external emission of the toxic vapours of the solvents.
d - rinsing of the reactor and loading for the polymerization. The load formulation was the following:
   - VCM: 45,000 Kg.
   - H₂O: 60,750 Kg.
   - Primary suspending agent of the partially hydrolyzed polyvinylic alcohol type, with hydrolysis degree equal to 72 % OH and viscosity of the 4% water solution equal to 7 cP: 27.0 Kg.
   - Secondary suspending agent of the partially hydrolyzed polyvinylic alcohol type, with hydrolysis degree equal to 55% OH: 27.0 Kg.
   - Lauroilperoxide-type catalyst: 22.5 Kg.
   - Reaction temperature: 70°C.

The reactor was equipped with a reflux-condenser fixed to the upper part of said reactor and connected to it by means of 12" pipes. The agitation system was based on 2 breakwaters and of 3 levels of agitation.

After a conversion equal to about 85% (Dp pressure 0,7 - 1 bar), the reactor was degasified and the mass in suspension transferred to the collection tanks.

During the transfer the slurry was filtered through a large filter blocking scale larger than 5 mm in diameter.

The statistical data on the cleaning of the reactor and on the collection of scale, on this specific and difficult type of PVC-S, are the following:
1 - scale collection in the transfer filter: about 80 Kg./batch, contaminated by layers of dark brown-black residues which detached after each polymerization batch.
2 - at the opening of the man-hole after 20 batches (loads) the reactor had the following problems:
   a) reflux condenser with visible deposits in the lower jacket wall and with deposits starting to form on the inner walls.
   b) very hard deposits, 4 mm thick, on the pipes connecting the reactor with the condenser.
   c) rather clean inner walls of the reactor, but with considerable deposits on the waterline, i.e. the liquid-gas interface during the reaction.
   d) a great layer of deposits on the shaft and in correspondence of the 3 agitation levels, weighting about 90 Kg.
   e) other blocks of deposits in correspondence of the supports for the side breakwaters.
   f) considerably clogged security valves and blowout disks, which had to be cleaned or replaced.

In order to remove these deposits and to restore the reactor to the normal conditions of polymerization, enormous amounts of labour were required and industrial production losses were recorded.

From an ecological point of view, the user had to dispose of large amounts of degraded PVC deposits which were polluted by the antifouling agent used.

On a reactor of such dimensions, formulations and productive cycles, a vapour spray system was installed for the antifouling agent prepared as described in example 1.

The treatment was carried out in the following way:
- 6 LT. of non polluting antifouling agent in a water solution, prepared as described in example 1, were loaded in a stainless steel barrel having a 15 LT. volume and pressurized with nitrogen having a pressure of 15 bar.
- Vapour at a pressure of about 8 bar is passed through a 2" pipe entering the head of the reactor.
- After 30 seconds of vapour inflow, the solution previously stored in the barrel is introduced in the same pipe.

In this way in only 1 seconds the antifouling agent water solution is sprayed inside the reactor and on all the parts connected to it.
- During the application, the inner walls are kept at the normal polymerization temperature (about 70 °C ± 8°C).
- By exploiting one of main characteristics of the new product, and namely that of coagulating at pH lower than 11, 90% of the antifouling agent loaded coagulates and precipitates together with the condensed vapour and may therefore be removed from the reactor and sent to the recovery set without causing any type of pollution.
- During the discharge, only 200 LT. of water are necessary for rinsing the vessel before starting the polymerization process; even this rinsing water is subjected to the treatment and reclaim together with the previously mentioned mother liquors.
- After this application, the inner walls of the reactor appear as bright stainless steel walls having a pale brown, extremely thin, uniform and scratch-resistant film.

By using this application technique after each load, it has been possible to reach 1000 consecutive loads carrying out only periodical inspections every 50 loads.

The data acquired were the following:
a) Scale collected in the slurry transfer filter (after each batch):
   - Kg. 10, having a very pale colour and with no trace of contamination.
b) After 50 batches (first inspection):
   - Perfectly clean reflux-condenser and connection lines.
   - Perfectly clean agitators and breakwaters with minimum traces (a few grammes) of PVC on the junction bolts.
   - Perfectly clean inner parts of the reactor, having only a few deposits on the waterline.
c) Without having been cleaned, the reactor was closed and further 250 batches were carried out, observing the reactor's heat exchange capacity, since the latter is the most sensitive parameter to the effects of the deposits on the walls of the reactor.

After 250 batches, the inspection only revealed a slight worsening of the problems described in A and it was therefore decided to proceed for an indefinite period.

After 1000 batches it was decided to interrupt the test and carry out a check-up of all the internal parts.

The small deposits on the agitator and on the blades were then removed, and the thermocouples cleaned, and, surprisingly, it was discovered that the upper parts of the reactor, the blowout disks, the relief valves, charge lines, reflux-condenser and so on, were perfectly clean and required no intervention.

It must be added that with this new antifouling agent, water at a high pressure was sufficient for cleaning, without requiring teams of specialized workmen to go inside the reactor in order to carry out this difficult and dangerous cleaning operation.

After these results, which classify the product as at least 20 times better than the standard antifouling agent used on other similar reactors of the same plant, it can be concluded that the new non polluting agent (as from example 1) had definitively solved the environmental and productive problems lamented by this plant.

### Example 3: Tests on the polymerization of Acrylo styrene butadiene (ABS) latexes.

In an 28 m³ industrial reactor, made in stainless steel iron, equipped with heating jacket, with two breakwaters and with an impeller-type agitator, a polymerization in acrylonitrile styrene butadiene emulsion is carried out in order to produce the basic latex for the production of ABS copolymers.

The load formulation normally utilized is the following:
- Filling of the reactor: 80%
- Water load: 65%
- Butadiene load: 24.5%
- Styrene load: 7%
- Acrylonitrile load: 3.5%
- Catalyst: Persulphate
- Emulsifier: Resinous soap
- Reaction temperature: 70 - 90°C

Normally, with no preventive antifouling treatment, the productive cycle must be interrupted only after about 8 polymerization loads, due to the formation of consistent polymer deposits on the inner parts of the reactor (walls, agitator and waterbreaks).

Such deposits reduce heat exchange between the reacting bulk and the jacket, making it impossible to control the polymerization temperature.

The reactor must therefore be washed with toluene at 100°C for 18 hours in order to eliminate such deposits.

In this polymerization process, the new non polluting antifouling agent produced according to example 1 was experimented, applying 1.5 LT. of it as previously described in example 2.

By using this application technique after each load, it has been possible to reach 50 consecutive loads, only making periodical inspections every 15 loads.

The results obtained were the following:
- After 15 loads: No formation of deposits.
- After 30 loads: Formation of small deposits localised only in some areas of the inner walls of the reactor.
- After 45 loads: More consistent deposits in some areas of the reactor and of the waterline which however did not compromise the normal work procedure of the reactor.
- After 50 loads: The reactor was washed according to the normal procedure.

Moreover, it was proved that the application of the antifouling agent did not even minimally influence the qualitative characteristics of the finished product.

### Example 4: Tests on the polvmerization in High Impact Polystyrene Suspension.

In a 45 m³, industrial reactor, made in stainless steel, equipped with heating jacket, with two breakwaters and an impeller-type agitator, a polymerization in HIPS High Impact (Shockproof) polystyrene suspension was carried out.

The load formulation normally utilized is the following:
- Filling of the reactor: 32 tons.
- Water/Styrene+Polybutadiene ratio: 0.8: 1.0
- Suspending agent of the polyvinylic alcohol type: 0.15%
- Polymer composition: 90% Styrene/10% Polybutadiene
- Polymerization temperature: 120°C - 160°C

Normally, with no preventive antifouling treatment, the productive cycle must be interrupted after about 40 polymerization loads, due to the formation of consistent polymer deposits localised in particular on the top of the surge tank, on the shaft of the agitator and on the breakwaters.

Such deposits not only reduce the heat exchange between the reacting bulk and the jacket, making it impossible to control the polymerization temperature, but also compromise the efficiency of the agitation and therefore the stability of the suspension.

Therefore, the reactor must be washed with a solvent (toluene or ethyl benzol) at about 100°C for 20 hours, in order to eliminate the deposits.

In this polymerization process, the new non polluting antifouling agent produced according to example 1 was experimented, applying 2.5 LT. of it as previously described in Example 2. By using this application technique after each load, in the same time interval and with the same number of loads, no deposits on the inner parts which required washing with solvents were detected.

Therefore, 150 loads were carried out before having to wash the vessel.

Moreover, it was proved that by applying the antifouling agent, the qualitative characteristics of the finished product did not even minimally change.

### Example 5: Tests on the polymerization in styrene and diethylbenzol emulsion.

In a 10 m³ industrial reactor, made in stainless steel, equipped with heating jacket, with two breakwaters and an impeller-type agitator, a polymerization in styrene and diethylbenzol emulsion was carried out in order to produce ion exchange resins.

The load formulation normally utilized is the following:
- Filling of the reactor: 85%
- Water/Monomers ratio: 1.0: 1.0 in weight
- Catalyst: Organic peroxide
- Emulsifier: Cellulose compounds
- Monomers composition: 50% Styrene/50% Diethylbenzene
- Polymerization temperature: 65 - 85°C

Normally, with no preventive antifouling treatment, the productive cycle must be interrupted after about 6-7 polymerization loads due to the formation of consistent polymer deposits on the inner parts of the reactor (walls, agitator and breakwaters).

Such deposits reduce heat exchange between the reacting bulk and the jacket, making it impossible to control the polymerization temperature and to modify the fluid dynamic conditions of the agitation.

The reactor must therefore be washed with toluene at 100°C for 10 hours in order to eliminate such deposits.

In this polymerization process the new non polluting antifouling agent produced according to example 1 was experimented, applying 1.0 LT of it as previously described in example 2.

By using this application technique after each load, no formation of deposits was detected for a productive cycle of at least 50 consecutive loads, and without influencing the quality of the finished product. At the end of the experimentation, normal production was carried out without an antifouling treatment, and an immediate formation of deposits was detected.

### Example 6: Tests on the polymerization of α-Methylstyrene (α-SAN) emulsion.

In a 25 m³ industrial reactor, made in stainless steel, equipped with heating jacket, with two breakwaters and an impeller-type agitator, a polymerization of α-Methylstyrene emulsion in order to produce α-SAN was carried out.

The load formulation normally utilized is the following:
- Filling of the reactor: 80%
- Water load: 74%
- α-Methylstirene load: 26%
- Catalyst: Common hydroperoxide
- Emulsifier: Resinous soap
- Reaction temperature: 70 -90°C

Normally, with no preventive antifouling treatment, the productive cycle must be interrupted after about 20 polymerization loads, due to the formation of consistent polymer deposits localised in particular on the agitator shaft and on the breakwaters.

These deposits modify the fluid dynamic conditions of the agitation, and therefore the production must be interrupted, and the reactor washed with toluene.

In this polymerization process, the new non polluting antifouling agent produced according to example 1 was experimented, applying 1.5 LT of it, as previously described in example 2.

By using this application technique after each load, no inner deposits requiring the washing with solvent were detected, in the same interval of time and with the same number of loads.

Moreover, it was proved that the application of the antifouling agent did not minimally influence the qualitative characteristics of the finished product.

## Claims

1. An antifouling material to be applied as a protective coating of the internal walls of a polymerization reactor, having naphthenic molecules of aromatic structure with radicals positioned from 1 to 8, according to the following arrangement: 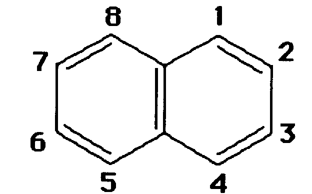 comprising in at least one of these radicals, at least one sulphur atom (S) and wherein the antifouling material contains sulphur higher than 0,25% in weight,
and wherein in that said radicals containing a sulphur atom, occupy at least one of positions: 2, 3 and 4 of the following structure: 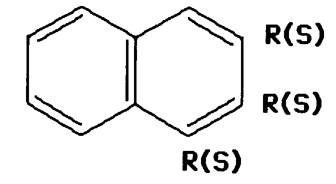 where R(S) represents a radical containing a sulphur atom (S), in any of positions 2,3 or 4, or also in more than one of said positions and wherein said radical also has at least one sodium atom or other salty alkali metal
- R (S, O, Na)
characterized in that in said radical there is the presence of a methylenic group (CH2) and/or a binder group (CH)-, said radical assuming the following structure:
-CH₂SOₙNa
where "n" is variable from 2 to 3, and/or
the following structure: 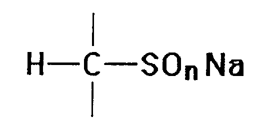 said radical being destined to bind two identical and opposed naphthenic aromatic groups where "n" is variable from two to three, and wherein:
the feature of its structure is proportioned and limited in order that:
- in the absence of oxygen it appears in the form of a transparent clear color, and if:
- in the presence of oxygen, it becomes a bluish or dark color, and if:
- the contact with the oxygen is interrupted, it reverts to its original aspect.

2. An antifouling material according to claim 1, characterized in that in said radical there is the presence of a binder group **(CH)**, said radical assuming the following structure: 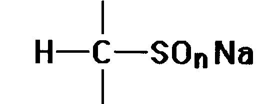 said radical being destined to bind two identical and opposed naphthenic aromatic groups where "n" is variable from two to three.

3. An antifouling material according to claims 1 and 2, characterized in that the naphthenic structure has a radical (OH), in position 1 according to the following structure: 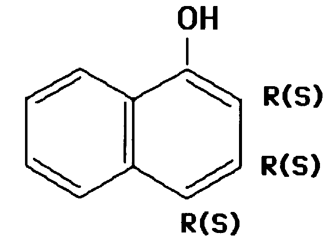 positions 2, 3 or 4 being able to be occupied by a radical containing at least one sulphur atom and positions 2 or 4 being able to constitute intermediate binder bridge between two identical and opposed naphthenic structures.

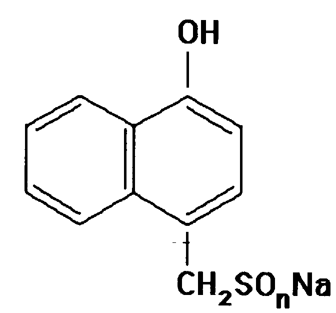

4. An antifouling material according to the preceding claims, characterized in that it has at least one molecule having the following structure: 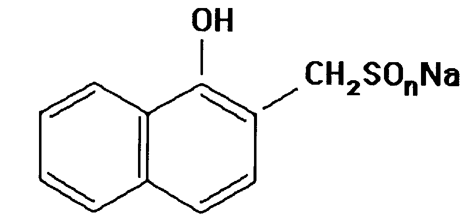 where "n" is variable from two to three.

5. An antifouling material according to the preceding claims, characterized in that it has at least one molecule having the following structure: 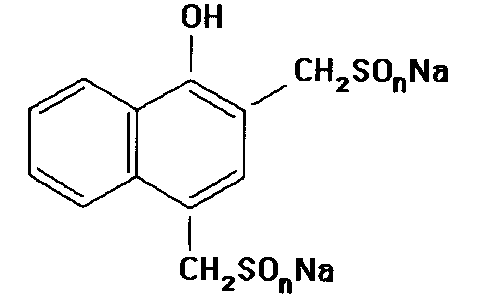 where "n" is variable from two to three.

6. An antifouling material according to the preceding claims, characterized in that it has at least one molecule having the following structure: 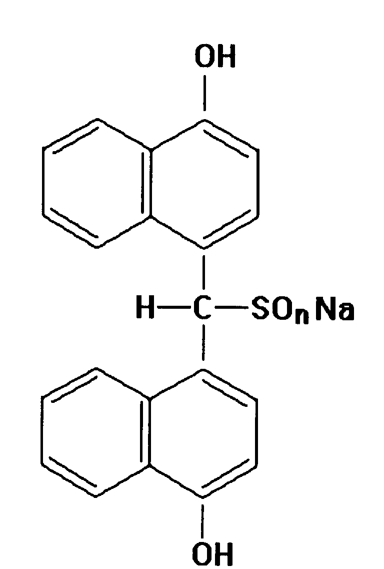 where "n" is variable from two to three.

7. An antifouling material according to the preceding claims, characterized in that it has at least one molecule having the following structure: 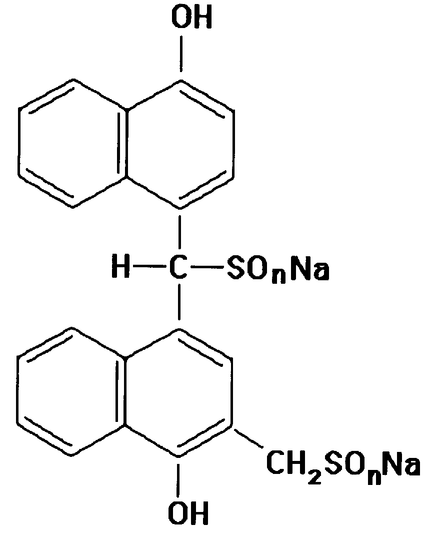 where "n" is variable from two to three.

8. An antifouling material according to the preceding claims, characterized in that it has at least one molecule having the following structure: 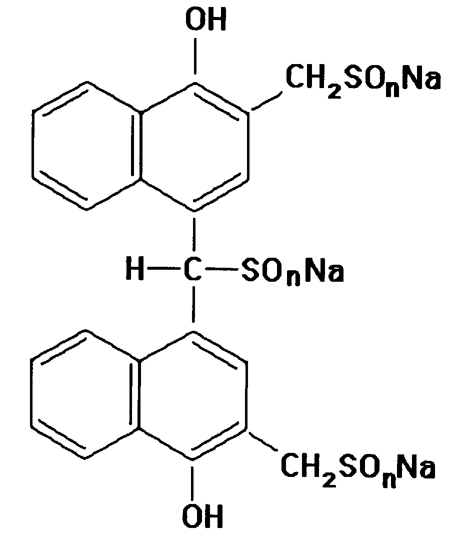 where "n" is variable from two to three.

9. An antifouling material according to the preceding claims, characterized in that it contains hydrosulphite.

10. An antifouling material according to the preceding claims, characterized in that it contains sodium hydrosulphite.

11. An antifouling material according to the preceding claims characterized in that it contains bisulphite.

12. An antifouling material according to the preceding claims characterized in that it contains sodium bisulphite.

13. An antifouling product according to the preceding claims, characterized in that it is contained in air tight containers, impermeable to oxygen.

14. An antifouling product according to the preceding claims, characterized in that it is contained in containers pressurized with inert gas.

15. An antifouling product according to the preceding claims, characterized in that it is contained in containers pressurized with nitrogen.

16. An antifouling product according to the preceding claims, characterized in that it is contained in polyethylentherephthalate plastic material (PET) containers.

17. An antifouling product according to the preceding claims, characterized in that it is contained in bottles of polyethylentherephthalate plastic material.

## Patentansprüche

1. Ein Fäulnisschutzmaterial zur Verwendung als Schutzüberzugsschicht für die Innenwände eines Polymerisations-Reaktors, mit Naphthenmolekülen aromatischer Struktur mit den Radikalen 1 bis 8 in der folgenden Anordnung: 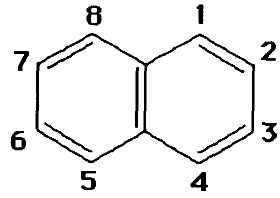 wobei in mindestens einem dieser Radikale mindestens ein Schwefelatom (S) vorhanden ist und das Fäulnisschutzmaterial mehr als 0,25 Gewichts-% Schwefel enthält,
und wobei diese besagten Radikale, die ein Schwefelatom enthalten, mindestens eine der Positionen: 2, 3 und 4 der folgenden Struktur einnehmen: 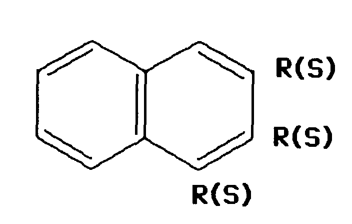 wobei R(e) eine Radikal darstellt, das ein Schwefelatom (S) enthält, in einer beliebigen der Positionen 2,3 oder 4, oder auch in mehr als einer der besagten Positionen
und wobei besagtes Radikal auch mindestens ein Natriumatom oder eins eines anderen salzigen Alkalimetalls
- R (S, O, Na)
enthält, gekennzeichnet dadurch, daß in besagtem Radikal eine Methylengruppe (CH2) und/oder eine Bindemittelgruppe (CH) vorhanden ist, wobei besagtes Radikal folgende Struktur annimmt:
- CH2SOnNa
wobei "n" veränderlich von 2 bis 3 ist, und/oder die folgende Struktur: 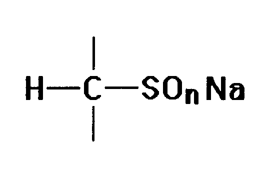 wobei besagtes Radikal dazu bestimmt ist, zwei identische und entgegensetzte aromatische Naphthengruppen zu binden, wobei "n" veränderlich von zwei bis drei ist,
und wobei:
das Merkmal der Struktur proportioniert und begrenzt ist, damit:
- es bei Sauerstoffabwesenheit in der Form einer transparenten klaren Farbe erscheint, und:
- es bei Anwesenheit von Sauerstoff bläuliche oder dunkle Farbe annimmt, und wenn:
- der Kontakt mit dem Sauerstoff unterbrochen wird, es zu seinem ursprünglichen Aussehen zurückkehrt.

2. Ein Fäulnisschutzmaterial nach Anspruch 1, gekennzeichnet dadurch, daß in besagtem Radikal eine Bindemittelgruppe (CH) vorhanden ist, wobei besagtes Radikal folgende Struktur annimmt: 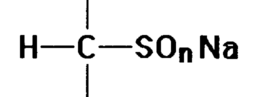 wobei besagtes Radikal dazu bestimmt ist, zwei identische und entgegensetzte aromatische Naphthengruppen zu binden, wobei "n" veränderlich von zwei bis drei ist.

3. Ein Fäulnisschutzmaterial nach Anspruch 1 und 2, gekennzeichnet dadurch, daß die Naphthenstruktur ein Radikal hat (OH), in Position 1 gemäß der folgenden Struktur: 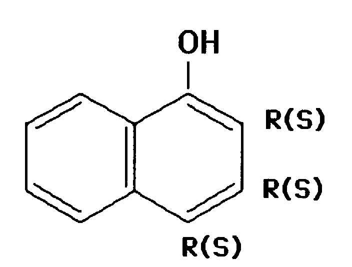 wobei die Positionen 2, 3 oder 4 von einem Radikal besetzt werden können, das mindestens ein Schwefelatom enthält, und wobei die Positionen 2 oder 4 eine Bindemittel-Brücke zwischen zwei identischen und entgegensetzten Naphthenstrukturen bilden können.

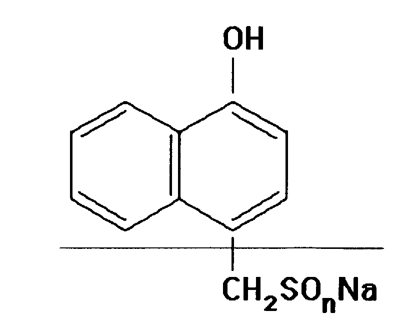

4. Ein Fäulnisschutzmaterial gemäß den vorherigen Patentansprüchen, gekennzeichnet dadurch, daß es mindestens ein Molekül mit der folgenden Struktur hat: 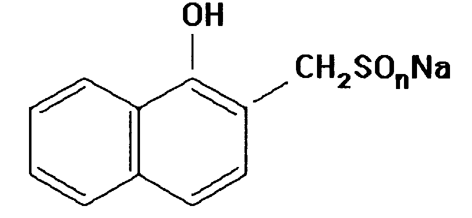 wobei "n" veränderlich von zwei bis drei ist.

5. Ein Fäulnisschutzmaterial nach den vorherigen Patentansprüchen, gekennzeichnet dadurch, daß es mindestens ein Molekül mit folgender Struktur hat: 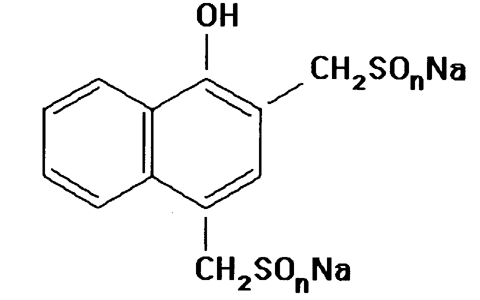 wobei "n" veränderlich von zwei bis drei ist.

6. Ein Fäulnisschutzmaterial nach den vorherigen Patentansprüchen, gekennzeichnet dadurch, daß es mindestens ein Molekül mit folgender Struktur hat: 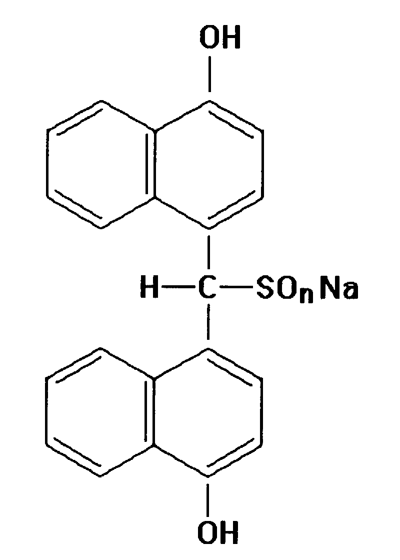 wobei "n" veränderlich von zwei bis drei ist.

7. Ein Fäulnisschutzmaterial nach den vorherigen Patentansprüchen, gekennzeichnet dadurch, daß es mindestens ein Molekül mit folgender Struktur hat: 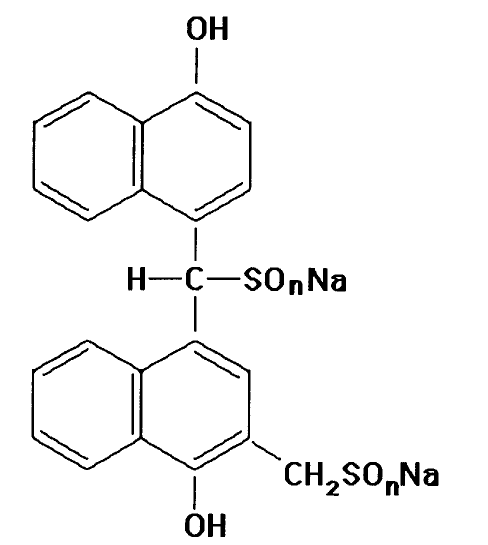 wobei "n" veränderlich von zwei bis drei ist.

8. Ein Fäulnisschutzmaterial nach den vorherigen Patentansprüchen, gekennzeichnet dadurch, daß es mindestens ein Molekül mit folgender Struktur hat: 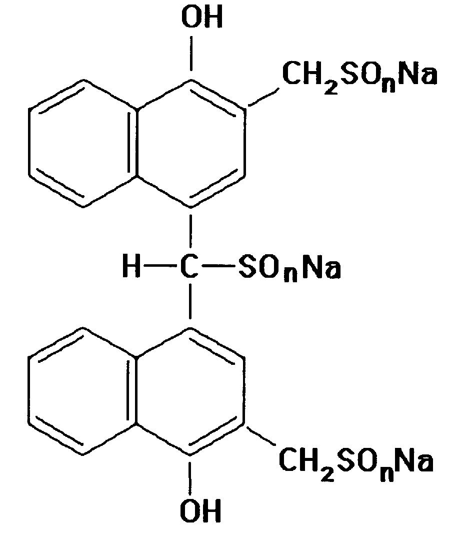 wobei "n" veränderlich von zwei bis drei ist.

9. Ein Fäulnisschutzmaterial nach den vorherigen Patentansprüchen, gekennzeichnet dadurch, daß es Hydrosulfit enthält.

10. Ein Fäulnisschutzmaterial nach den vorherigen Patentansprüchen, gekennzeichnet dadurch, daß es Natriumhydrosulfit enthält.

11. Ein Fäulnisschutzmaterial nach den vorherigen Patentansprüchen gekennzeichnet dadurch, daß es Hydrogensulfit enthält.

12. Ein Fäulnisschutzmaterial nach den vorherigen Patentansprüchen gekennzeichnet dadurch, daß es Natriumhydrogensulfit enthält.

13. Ein Fäulnisschutzprodukt nach den vorherigen Patentansprüchen, gekennzeichnet dadurch, daß es in einem luftdichten und sauerstoffundurchlässigen Behälter enthalten ist.

14. Ein Fäulnisschutzprodukt nach den vorherigen Patentansprüchen, gekennzeichnet dadurch, daß es in Behältern enthalten ist, die mit Edelgas unter Druck gesetzt sind.

15. Ein Fäulnisschutzprodukt nach den vorherigen Patentansprüchen, gekennzeichnet dadurch, daß es in Behältern enthalten ist, die mit Stickstoff unter Druck gesetzt sind.

16. Ein Fäulnisschutzprodukt nach den vorherigen Patentansprüchen, gekennzeichnet dadurch, daß es in Polyethylentherephthalatkunststoff-(PET)-Behältern enthalten ist.

17. Ein Fäulnisschutzprodukt nach den vorherigen Patentansprüchen, gekennzeichnet dadurch, daß es in Flaschen aus Polyethylentherephthalat-Kunststoff enthalten ist.

## Revendications

1. Matériel antipourriture à appliquer comme revêtement protectif des murs internes d'un réacteur de polymérisation, ayant des molécules naphtheniques de structure aromatique avec les radicaux positionnés de 1 à 8, selon l'arrangement suivant: 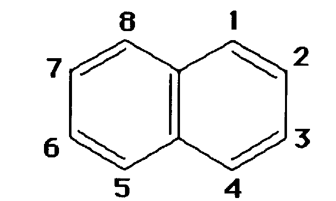 comprenant dans au moins un de ces radicaux au moins un atome de soufre (S) et dans lequel le matériel antipourriture contient du soufre supérieur à 0,25% de poids,
et dans lequel lesdits radicaux contenant un atome de soufre occupent au moins une des positions: 2, 3 et 4 de la structure suivante: 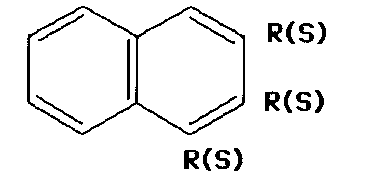 où R(S) représente un radical contenant un atome de soufre (S), dans n'importe quelle des positions 2,3 ou 4, ou également dans plus qu'une desdites positions
et dans lequel ledit radical également a au moins un atome de sodium ou d'un autre métal d'alcali salé
- R (S, O, Na)
caractérisé en ce que dans ledit radical il y a la présence d'un groupe méthylénique (CH2) et/ou d'un groupe relieur (CH), où ledit radical assume la structure suivante:
- CH2SOnNa
où "n" est variable de 2 à 3, et/ou
la structure suivante: 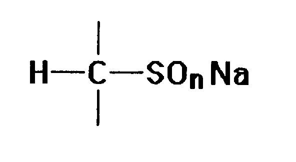 ledit radical étant destiné à lier deux groupes naphthéniques aromatiques identiques et opposés où "n" est variable de deux à trois, et dans lequel:
la caractéristique de sa structure est proportionnée et limitée afin que:
- en absence d'oxygène il apparaît dans la forme d'une couleur claire transparente, et:
- en présence d'oxygène, il assume une couleur bleuâtre ou sombre, et si:
- le contacte avec l'oxygène est interrompu, il revient à son aspect original.

2. Matériel antipourriture selon la revendication 1, caractérisé en ce que dans ledit radical il y a la présence d' un groupe relieur (CH), ledit radical assumant la structure suivante: 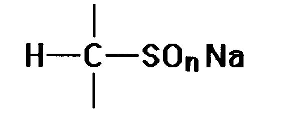 ledit radical étant destiné à lier deux groupes naphthéniques aromatiques identiques et opposés où "n" est variable de deux à trois.

3. Matériel antipourriture selon les revendications 1 et 2, caractérisé en ce que la structure naphthénique a un radical (OH) en position 1 selon la structure suivante: 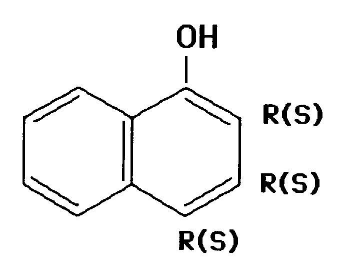 les positions 2, 3 ou 4 pouvant être occupées par un radical contenant au moins un atome de soufre et les positions 2 ou 4 pouvant constituer un pont relieur intermédiaire entre deux structures naphthéniques identiques et opposées.

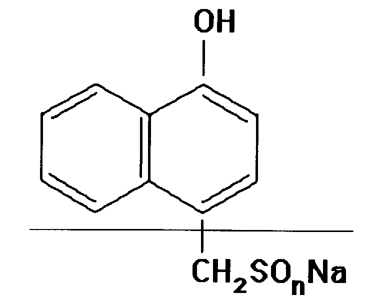

4. Matériel antipourriture selon les revendications précédantes, caractérisé en ce qu'il a au moins une molécule ayant la structure suivante: 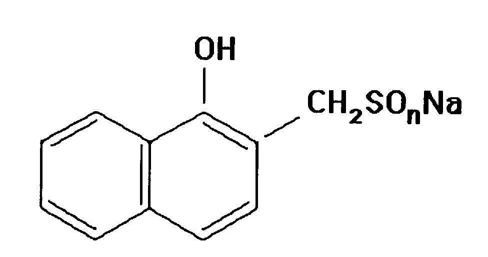 où "n" est variable de deux à trois.

5. Matériel antipourriture selon les revendications précédantes, caractérisé en ce qu'il a au m oinsune molécule ayant la structure suivante: 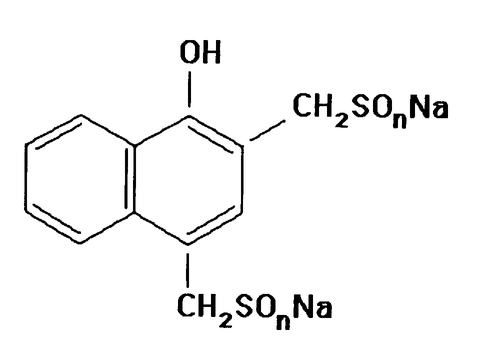 où "n" est variable de deux à trois.

6. Matériel antipourriture selon les revendications précédantes, caractérisé en ce qu'il a au moins une molécule ayant la structure suivante: 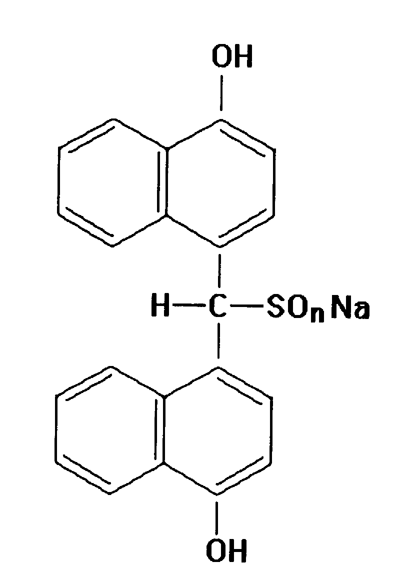 où "n" est variable de deux à trois.

7. Matériel antipourriture selon les revendications précédantes, caractérisé en ce qu'il a au moins une molécule ayant la structure suivante: 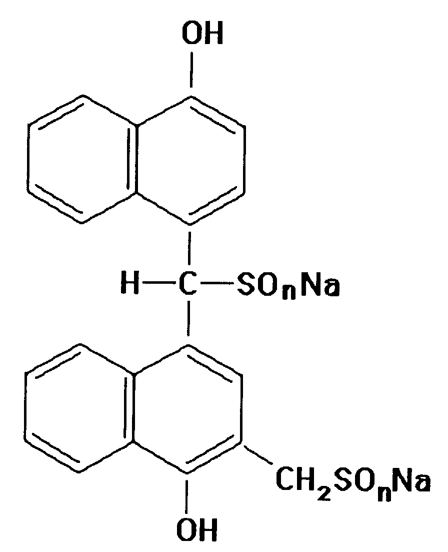 où "n" est variable de deux à trois.

8. Matériel antipourriture selon les revendications précédantes, caractérisé en ce qu'il a au moins une molécule ayant la structure
suivante: 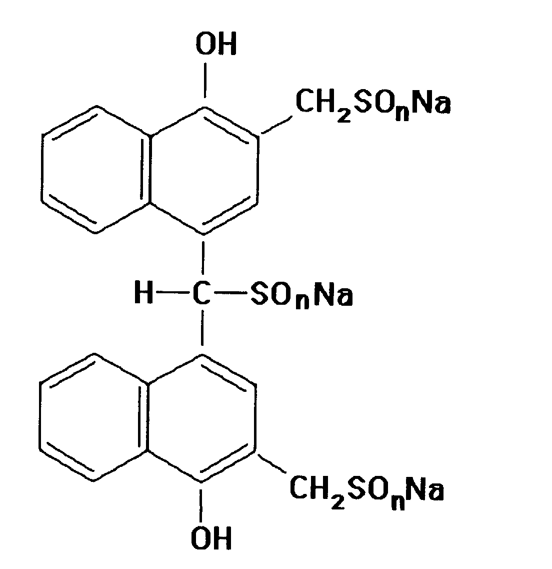 où "n" est variable de deux à trois.

9. Matériel antipourriture selon les revendications précédantes, caractérisé en ce qu'il contient de l'hydrosulphite.

10. Matériel antipourriture selon les revendications précédantes, caractérisé en ce qu'il contient de l'hydrosulphite de sodium.

11. Matériel antipourriture selon les revendications précédantes caractérisé en ce qu'il contient du bisulphite.

12. Matériel antipourriture selon les revendications précédantes caractérisé en ce qu'il contient du bisulphite de sodium.

13. Un produit antipourriture selon les revendications précédantes, caractérisé en ce qu'il est contenu dans des récipients imperméables à l'air, imperméables à l'oxygène.

14. Un produit antipourriture selon les revendications précédantes, caractérisé en ce qu'il est contenu dans des récipients pressurisés avec du gaz inerte.

15. Un produit antipourriture selon les revendications précédantes, caractérisé en ce qu'il est contenu dans des récipients pressurisés avec de l'azote.

16. Un produit antipourriture selon les revendications précédantes, caractérisé en ce qu'il est contenu dans des récipients en matériel plastique de polyéthylenthérephthalate (PET).

17. Un produit antipourriture selon les revendications précédantes, caractérisé en ce qu'il est contenu dans des bouteilles de matériel plastique de polyéthylenthérephthalate .
